# EUROPEAN PATENT APPLICATION

(11) **EP 2 210 934 A1**
(43) Date of publication of application: **28.07.2010**
(21) Application number: 10151468.5
(22) Date of filing: 22.01.2010
(51) Int. Cl.: C12M 1/107, C12P 5/02

(54) **Method and device for fermenting wet biomass**

(30) Priority: 22.01.2009 NL 1036447
(71) Applicant: Host B.V., 7521 PS Enschede (NL)
(72) Inventor: Kleizen, Roy Johannes Franciscus, 7587 SB, De Lutte (NL); Klein Teeselink, Hermanus, 7548 PJ, Boekelo (NL)
(74) Representative: Schumann, Bernard Herman Johan

(57) **Abstract**

The invention relates to a method for fermenting wet biomass, wherein use is made of a device (1) according to the invention, comprising:
- a tank (2) provided with an inlet (3) for admitting material for fermenting and provided with an outlet (4) for discharging fermented material;
- a discharge (5) in gas throughflow connection with the tank for discharging produced biogas; and
- stirring means (6) for stirring and mixing material present in the tank,
wherein
material present in the tank is stirred in a manner such that the mixing is incomplete and multiple zones (2a,2b,2c) form in the tank with dry matter contents differing from each other.

## Description

### Field of the invention

The invention relates to a method for fermenting wet biomass, wherein use is made of a device comprising:
- a tank provided with an inlet for admitting material for fermenting and provided with an outlet for discharging fermented material;
- a first discharge for discharging produced biogas; and
- stirring means for stirring and mixing material present in the tank.

The invention also relates to a device for fermenting wet biomass according to such a method. This relates particularly to the fermenting of manure, but optionally also sewage sludge, crop residues, food waste and other organic-biological material.

### Background of the invention

Known is the anaerobic fermentation of biomass wherein complex organic material is converted by micro-organisms and decomposed into inorganic compounds. The following steps can be distinguished in the overall process:
- hydrolysis, wherein proteins, carbohydrates and fats are converted into amino acids, sugars, higher fatty acids and alcohols;
- fermentation, wherein intermediary products such as propionate, butyrate are formed;
- acetogenesis, wherein hydrogen, carbon dioxide and acetate are formed; and
- methanogenesis, wherein methane and carbon dioxide are formed.

Anaerobic fermentation takes place in a closed tank. A stirring means is arranged in the tank for homogenizing the material. In the case of liquid, readily fermentable products it is for instance possible to suffice with a simple submersion mixer. In the case of materials which are difficult to process and ferment it is for instance possible to opt for a paddle stirrer or a plurality of mixers together with a ground scraping means in order to prevent floating layers and/or sedimentation layers. Mixing is also possible by means of pumping round or by introducing gas. The tank is generally of steel or concrete. A steel tank can be constructed more quickly but will usually not be strong enough to support the necessary technical installations such as the stirring means or the pump, so that other provisions must be made for this purpose. A concrete tank can be integrated with the foundation and will generally provide sufficient support strength for said technical installations.

The most important factors and process parameters in anaerobic fermentation of biomass are:
- scale; wherein a distinction can be made between smaller farm fermenters (tank volume of for instance 1200 m³), wherein only manure and/or agricultural crops from a farm itself are processed and the fully fermented material, the digestate, is utilized again on the farmer's own land, larger farm fermenters (tank volume of for instance 2000 m³), wherein materials from third parties are also supplied and processed, and central, regional or industrial fermenters (tank volume of for instance 2 x 1800 m³), wherein materials are supplied from various locations;
- type of installation; wherein a distinction can be made between (upright) fully mixed fermenters (CSTR, more suitable at lower dry matter contents) and (horizontal) plug-flow fermenters (more suitable at higher dry matter contents);
- type of fermentation; wherein a distinction can be made between manure fermentation of manure alone and co-fermentation, wherein animal fertilizers are fermented together with other organic products (co-substrates);
- feed regime; wherein a distinction can be made between fermenters with a continuous, semi-continuous or quasi-continuous supply and discharge, and discontinuous or 'batch' fermenters;
- type of feed; the quantity and the type of the supplied material for fermenting (manure, sewage sludge, organic waste, dump waste, industrial waste; energy corn, elephant grass and/or other organic-biological material);
- dry matter content; the percentage (by weight) of the undissolved matter, wherein a distinction can be made between wet biomass (dry matter content < 20%) and dry biomass (dry matter content > 20%);
- contamination; physical (sand, stones and the like) or substances which affect the biological process (for instance antibiotics and cleaning agents);
- pretreatment; removal of physical contaminants such as sand and stones, reducing in size, wet or dry separation, mixing and homogenizing, pasteurizing and sterilizing;
- mixing; wherein a distinction can be made between fermenters with a continuous or discontinuous mixing, and fermenters with biogas mixing or mechanical mixing;
- stirring; in order to ensure that the material in the tank can be stirred and mixed the dry matter content must not be too high;
- number of phases or processing steps; wherein a distinction can be made between a single-phase system, wherein the whole process takes place in one tank, and a two-phase system wherein the hydrolysis takes place in a first tank and the methanogenesis in a second tank;
- residence time; the (average) time that the material is in the tank;
- temperature; wherein a distinction can be made between cold, unheated or psychrophilic (10-20□C), mesophilic (30-40□C) and thermophilic (50-60□C);
- acidity; pH value;
- formation of process-inhibiting substances (for instance ammonia);
- digestate; the quantity and type of the remaining fermented material; and
- further treatment; such as separating, composting, drying, further fermentation and pasteurization of the digestate, or the purifying of the biogas or removal of the carbon dioxide therefrom.

The produced biogas has the main constituents methane (60-70%) and carbon dioxide (30-40%); said percentages apply when the fermentation is effective. The biogas can be used for the production of electrical energy and heat, part of which can in turn be used for the process itself. The formed methane can also be applied as car fuel or is mixed into the natural gas network. The digestate is used in agriculture as ground-improving fertilizer and for other purposes.

Advantages of anaerobic fermentation of organic-biological waste are the production of sustainable energy, the limitation of the methane emissions from waste and manure storage, the reduction of emissions to ground and surface water, the reduction of fertilizer usage, the re-use of waste products as valuable fertilizer, the killing of pathogens (sanitation) and weed seeds. There are on the other hand drawbacks such as the investment required and the costs to be incurred, the emissions of ammonia, the possibly necessary transport for supply and discharge of the required and the produced materials, and the possible health and safety risks.

The investment costs for an installation for anaerobic fermentation of biomass generally lie between 0.5 and 2.5 million euros. In an average case the operational costs of operation, maintenance and insurance amount to about 5% of the investment cost. Added to that are the labour costs and the costs for collection and delivery of manure and possible co-substrates. The efficiency of the installation is determined mainly by the biogas yields, the payment for the produced electricity, the cost price of co-substrates and the investment costs. The investment costs represent a significant cost overhead and threshold, especially in the case of larger installations. A significant part of these investment costs are related to the construction of the tank. The volume of the tank is determined by the size of the supply of material for fermenting and the (average) residence time in the tank. For a usual mesophilic process for the fermentation of wet biomass the residence time amounts to 15-40 days. In a farm fermenter the supply of material for fermenting is for instance 50 m³ per day. The minimum required tank size is then between 750 and 2000 m³. Such a voluminous and heavy tank is expensive and is constructed on-site, since transport from a construction site elsewhere is not feasible from an engineering and/or economic viewpoint.

There now exists a need for an improved method and device for fermenting wet biomass, wherein the investment costs, and preferably also the operational costs, are substantially lower than in usual installations and methods. The present invention provides a solution which meets this need.

### Summary of the invention

According to the invention wet biomass is fermented making use of a device comprising:
- a tank provided with an inlet for admitting material for fermenting and provided with an outlet for discharging fermented material;
- a first discharge in gas throughflow connection with the tank for discharging produced biogas; and
- stirring means for stirring and mixing material present in the tank,
**characterized in that** material present in the tank is stirred in a manner such that the mixing is incomplete and multiple zones form in the tank with dry matter contents substantially differing from each other. Multiple zones lying one above another can thus be formed, wherein due to sedimentation the dry matter content is lower in an upper-lying zone than the dry matter content in a lower-lying zone. The greater part of the dry matter and the active micro-organisms can then be situated in the lower-lying zone and the fermentation process can largely take place here, while the concentration of dry matter and micro-organisms in the upper-lying zone will be much lower. By now connecting the outlet to the upper-lying zone the flushing of micro-organisms will be relatively low. This flushing can be minimized still further by filtering discharged fermented material by means of filtering means provided for this purpose. The inlet is preferably also connected to the upper-lying zone. Said measures have a favourable effect on the overall fermenting process and the biogas yields, and reduce the necessary residence time. The required volume of the tank is hereby minimized, thereby decreasing the necessary investment and the operational costs.

Material present in the tank is preferably stirred and incompletely mixed in a manner such that the nature of the dry matter in a zone differs substantially from the nature of the dry matter in another zone. Preferably such that the nature of the dry matter in a lowermost zone is substantially sandy and/or stony. In an embodiment of the device according to the invention the sandy and/or stony dry matter can be discharged through a second discharge connected for this purpose to the lowermost zone. A pretreatment wherein sand and stones are removed is then unnecessary, and the digestate will be free of these physical contaminants.

The material present in the tank can be heated by means of heating means provided for this purpose. This increases the fermentation speed and reduces the necessary residence time. Material present in the tank is preferably heated to above 50□C. The fermentation speed can thus be maximized and the necessary residence time minimized, even such that, as is found in practice, it is possible to suffice in the case of a usual farm fermenter with a tank of only several hundred m³. In specific cases, when the supply of material for fermenting is relatively small, the required volume of the tank can even be 100 m³ or less. It then becomes possible to give the tank a relatively very light form, for instance in plastic. Owing to its relatively low weight, and particularly to its small volume, the tank can then be manufactured elsewhere and then be transported to the location of use. This all results in a considerable reduction in the necessary investment costs.

### Short description of the figures

The invention is elucidated hereinbelow on the basis of a number of exemplary embodiments of devices and methods for fermenting wet biomass according to the prior art and non-limitative exemplary embodiments of devices and methods according to the invention, shown more or less schematically in a number of figures. Herein:
- figure 1 shows a first known device with a continuously fed, fully stirred and mixed tank;
- figure 2 shows a second known device with a discontinuously fed tank;
- figure 3 shows a third known device with a continuously fed tank with a plug-flow; and
- figure 4 shows an exemplary embodiment of a device according to the invention with a continuously fed, stirred but incompletely mixed tank.

### Exemplary embodiments of a device and a method according to the invention

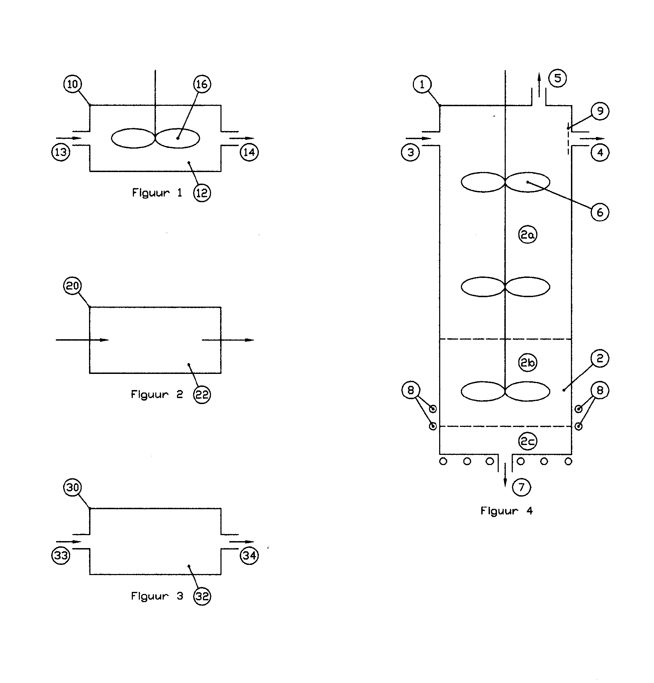

Figure 1 shows a first known device (10), a 'CSTR', with a continuously fed, fully stirred and mixed tank (12) provided with an inlet (13), an outlet (14) and stirring means (16). Such a device is more suitable for fermenting wet biomass which can be readily pumped and mixed. The dry matter content of the material for fermenting amounts generally to 6-20%, the temperature in the tank to 40-50□C, and the residence time, depending on the type of feed, to 20-80 days. The dry matter content of the digestate is roughly equal to the dry matter content of the material in tank (12), which remains roughly constant during the fermentation process.

Figure 2 shows a second known device (20), a 'batch fermenter', with a discontinuously fed tank (22). Tank (22) is loaded, closed, after which the fermentation takes place, and then unloaded. Such a device is more suitable for fermenting dry biomass, which is difficult to pump and mix. The dry matter content of the material for fermenting amounts generally to > 20%, the temperature in the tank to 40-50□C and the residence time, depending on the type of feed, to 20-80 days. The dry matter content of the material in tank (22) decreases during the fermentation process.

Figure 3 shows a third known device (30), a 'plug-flow fermenter' with a continuously fed horizontal tank (32) provided with an inlet (33) and an outlet (34). Such a device is suitable for fermenting both wet and dry biomass. The dry matter content of the material for fermenting amounts generally to 6-30%, the temperature in the tank to 40-50□C, and the residence time, depending on the type of feed, to 20-80 days. The dry matter content of the material in tank (22) decreases as it moves from inlet (33) to outlet (34).

The tank (12,22,32) of a known device (10,20,30) has a volume of for instance 1000-5000 m³ and is built on location. The costs for the construction of the tank form a substantial part of the investment cost and are relatively high.

Figure 4 shows an exemplary embodiment of a device (1) according to the invention with a continuously fed, stirred, but incompletely mixed tank (2). Such a device is particularly suitable for fermenting wet biomass with a dry matter content of 6-10% which can be readily pumped and mixed. Tank (2) is provided with an inlet (3), an outlet (4) and stirring means (6). The dry matter content of the material for fermenting amounts to for instance 8%. The volume of tank (2) is stirred and incompletely mixed in a manner such that, in the given example, three zones (2a,2b,2c) result. An upper-lying first zone (2a) comprises relatively little dry matter, for instance 4%, largely already fermented material, and relatively few micro-organisms. Stirring takes place such that no floating layers, for instance of crude cellulose, form in first zone (2a). A lower-lying second zone (2b) comprises a relatively large amount of dry matter, for instance 10%, largely material still to be fermented, and a relatively large number of micro-organisms.

The fermentation takes place mainly in second zone (2b). Inlet (3) and outlet (4) are connected to the upper-lying first zone (2a). The dry matter content of the discharged fermented material is roughly equal to the dry matter content of first zone (2a), which remains roughly constant during the fermentation process. The flushing of micro-organisms via outlet (4) is low. The flushing is minimized still further by filtering discharged fermented material by means of filtering means (9) provided for this purpose. Said measures have a favourable effect on the whole fermentation process and the biogas yield, and reduce the necessary residence time. The required volume of tank (2) is hereby minimized, thereby decreasing the necessary investments and the operational costs.

By stirring and incompletely mixing in a correct manner, a lowermost third zone (2c) is also formed having therein substantially settled physical contaminants such as sand and/or stones. This sand and/or these stones can be discharged via a second discharge (7) provided for this purpose. A pretreatment wherein sand and stones are removed is then unnecessary, and the digestate will be free of these physical contaminants.

The material present in the tank can be heated by means of heating means (8) provided for this purpose in the form of hot water pipes. This increases the fermentation speed and reduces the necessary residence time. The material present in tank (2) is for instance heated to 60□C. In the given example the fermentation speed is then found to be maximal, and the necessary residence time minimal, only 4-10 days depending on the type of feed. At a supply of material for fermenting of for instance 30-50 m³ per day a volume of tank (2) can then suffice of only about 150-600 m³. At an even smaller supply of for instance 10-20 m³ per day and a residence time of 4 days, such as in a relatively small farm fermenter for manure, it is even possible to suffice with a volume of tank (2) of only about 50-100 m³. Owing to its relatively small volume, the tank can then be manufactured elsewhere, for instance in plastic or steel, and then be transported to the location of use. This all results in a considerable reduction in the necessary investment costs relative to known devices.

It will be apparent that the invention is not limited to the given exemplary embodiments, but that diverse variants are possible within the scope of the invention.

## Claims

1. Method for fermenting wet biomass, wherein use is made of a device (1) comprising:
- a tank (2) provided with an inlet (3) for admitting material for fermenting and provided with an outlet (4) for discharging fermented material;
- a discharge (5) in gas throughflow connection with the tank for discharging produced biogas; and
- stirring means (6) for stirring and mixing material present in the tank,
**characterized in that**
material present in the tank is stirred in a manner such that the mixing is incomplete and multiple zones (2a,2b,2c) form in the tank with dry matter contents differing from each other.

2. Method as claimed in claim 1,
**characterized in that**
material present in the tank is stirred and incompletely mixed in a manner such that the average dry matter content in an upper-lying zone (2a) is substantially equal to, and the average dry matter content in a lower-lying zone (2b) is higher than the dry matter content of the discharged fermented material.

3. Method as claimed in claim 2,
**characterized in that**
at least substantially fermented material is discharged from the upper-lying zone.

4. Method as claimed in claim 2 or 3,
**characterized in that**
material for fermenting is admitted into the upper-lying zone.

5. Method as claimed in any of the claims 1-4,
**characterized in that**
discharged fermented material is filtered by means of filtering means (9) provided for this purpose.

6. Method as claimed in any of the claims 1-5,
**characterized in that**
material present in the tank is stirred and incompletely mixed in a manner such that the nature of the dry matter in a zone (2a,2b) differs substantially from the nature of the dry matter in another zone (2c), wherein the nature of the dry matter in a lowermost zone is substantially sandy and/or stony.

7. Method as claimed in claim 6,
**characterized in that**
the sandy and/or stony dry matter is discharged through a second discharge (7) connected for this purpose to the lowermost zone.

8. Method as claimed in any of the claims 1-7,
**characterized in that** material present in the tank is heated by means of heating means (8) provided for this purpose.

9. Device (1) for fermenting wet biomass, comprising:
- a tank (2) provided with an inlet (3) for admitting material for fermenting and provided with an outlet (4) for discharging fermented material;
- a first discharge (5) in gas throughflow connection with the tank for discharging produced biogas; and
- stirring means (6) for stirring and mixing material present in the tank,
**characterized in that**
the stirring means have a form such that during the stirring and mixing multiple zones (2a,2b,2c) are formed with dry matter contents differing substantially from each other.

10. Device as claimed in claim 9,
**characterized in that**
the stirring means have a form such that the average dry matter content in an upper-lying zone (2a) is substantially equal to, and the average dry matter content in a lower-lying zone (2b) is substantially higher than the dry matter content of the discharged fermented material.

11. Device as claimed in claim 10,
**characterized in that**
the outlet is connected to the upper-lying zone and/or that the inlet is connected to the upper-lying zone.

12. Device as claimed in any of the claims 9-11,
**characterized in that** the device also comprises filtering means (9) for filtering discharged fermented material.

13. Device as claimed in any of the claims 9-12,
**characterized in that**
the stirring means have a form such that the nature of the dry matter in a zone (2a,2b) differs substantially from the nature of the dry matter in another zone (2c), wherein the nature of the dry matter in a lowermost zone is substantially sandy and/or stony.

14. Device as claimed in claim 13,
**characterized in that**
the tank is provided with a second discharge (7) for discharging the sandy and/or stony dry matter, which second discharge is connected to the lowermost zone.

15. Device as claimed in any of the claims 9-14,
**characterized in that**
the device also comprises heating means (8) for heating material present in the tank.
